(19) Europäisches Patentamt
European Patent Office
Office européen des brevets

(11) **EP 2 841 207 B1**

(12) **EUROPEAN PATENT SPECIFICATION**

(45) Date of publication and mention
of the grant of the patent:
**11.01.2017   Bulletin 2017/02**

(21) Application number: **13721195.9**

(22) Date of filing: **23.04.2013**

(51) Int Cl.:
**B05B 17/00** *(2006.01)*      **B05B 17/06** *(2006.01)*

(86) International application number:
**PCT/US2013/037772**

(87) International publication number:
**WO 2013/163163 (31.10.2013 Gazette 2013/44)**

(54) **DELIVERY SYSTEM COMPRISING IMPROVED VOLATILE COMPOSITIONS**

ABGABESYSTEM MIT VERBESSERTEN FLÜCHTIGEN ZUSAMMENSETZUNGEN

SYSTÈME DE DISTRIBUTION CONTENANT DES COMPOSITIONS VOLATILES AMÉLIORÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **27.04.2012   US 201261639112 P**

(43) Date of publication of application:
**04.03.2015   Bulletin 2015/10**

(73) Proprietor: **The Procter & Gamble Company Cincinnati, OH 45202 (US)**

(72) Inventors:
• **JACKSON, Rhonda, Jean**
  **Cincinnati, Ohio 45239 (US)**
• **DIERSING, Steven, Louis**
  **Cincinnati, Ohio 45211 (US)**
• **GRUENBACHER, Dana, Paul**
  **Fairfield, Ohio 45014 (US)**
• **MORGAN III, George, Kavin**
  **Hamilton, Ohio 45011 (US)**
• **TURNER, Ronald, David**
  **Walton, Kentucky 41094 (US)**

(74) Representative: **Kellenberger, Jakob Procter & Gamble Services Co. Temselaan 100 1853 Strombeek-Bever (BE)**

(56) References cited:
**WO-A1-2013/105096      WO-A2-2012/101642
JP-A- 2008 168 222      US-A1- 2011 266 359
US-A1- 2011 268 605      US-B2- 8 877 139**

**Description**

FIELD OF THE INVENTION

The present invention relates to a delivery system comprising an improved volatile composition, such as a perfume composition. The delivery system comprises an electromechanical or electroacoustical actuator for emitting, in the form of a spray of fine particles, the improved volatile composition into the atmosphere.

BACKGROUND OF THE INVENTION

The emission or atomization of volatile compositions by formation of a fine spray is well known. Various attempts have been made to provide means and methods for liquid dispersion.

One atomizer spraying delivery system is shown by Humberstone et al, in U.S. Patent No. 5,518,179, which teaches a liquid droplet production apparatus comprising a membrane which is vibrated by an actuator which has a composite thin-walled structure, and is arranged to operate in a bending mode. Liquid is supplied directly to a surface of the membrane and sprayed therefrom in fine droplets upon vibration of the membrane.

U.S. Patent Nos. 5,297,734 and 5,657,926, of Toda, teach ultrasonic atomizing delivery systems comprising piezoelectric vibrators with a vibrating plate connected thereto. In U.S. Pat. No. 5,297,734, the vibrating plate is described as having a large number of minute holes therein for passage of the liquid.

U.S. Patent No. 6,378,780 teaches a method of dispensing a liquid formulation with uniform consistency over extended periods of time with liquid formulation having a viscosity below ten centipoise.

U.S. Patent Publication No. 2011/0266359 provides an ultrasonic micro-plug unit for emitting scented liquids into the air. The ultrasonic micro-plug unit has a plate with a plurality of perforations and a second plate with a plurality of micro-plus that mate with the perforations. Upon actuation of the piezoelectric element, the plugs separate from the perforations enabling the scented liquid to atomize into the air.

While a number of patents disclose means for atomizing liquids by piezoelectric actuators, there remains a need to provide a piezoelectric delivery system comprising a volatile composition formulated for such delivery system to improve scent longevity, perfume character, room fill, and/or functional benefits (e.g. odor elimination).

SUMMARY OF THE INVENTION

In one embodiment, there is provided a delivery system comprising: a volatile composition comprising from about 15% to about 50%, by weight of the volatile composition, of materials having a Kovat's Index greater than 1400, wherein the volatile composition is contained in a reservoir having a capillary element. The delivery system also comprises an ultrasonic micro-plug unit comprising a first plate comprising a plurality of perforations extending through the first plate; a second plate comprising a plurality of micro-plugs extending longitudinally from base end to a tip end, and configured to mate with at least one of the plurality of perforations; and a piezoelectric actuator in communication with one or more of the first and second plates; wherein the capillary element transports the volatile composition from the reservoir to the first plate, and wherein the piezoelectric actuator, upon receiving an electrical signal, displaces at least one micro-plug from a seated position in at least one of the perforations and atomizes the volatile composition.

In another embodiment, there is provided a delivery system comprising a volatile composition comprising greater than 50%, by weight of the volatile composition, of at least one FPC, wherein the volatile composition is contained in a reservoir having a capillary element. The delivery system also comprises an ultrasonic micro-plug unit comprising a first plate comprising a plurality of perforations extending through the first plate; a second plate comprising a plurality of micro-plugs extending longitudinally from base end to a tip end, and configured to mate with at least one of the plurality of perforations; and a piezoelectric actuator in communication with one or more of the first and second plates; wherein the capillary element transports the volatile composition from the reservoir to the first plate, and wherein the piezoelectric actuator, upon receiving an electrical signal, displaces at least one micro-plug from a seated position in at least one of the perforations and atomizes the volatile composition.

In yet another embodiment, there is provided a delivery system comprising a volatile composition comprising greater than 15%, by weight of the volatile composition, of materials having a boiling point greater than 250°C, wherein the volatile composition is contained in a reservoir having a capillary element. The delivery system also comprises an ultrasonic micro-plug unit comprising a first plate comprising a plurality of perforations extending through the first plate; a second plate comprising a plurality of micro-plugs extending longitudinally from base end to a tip end, and configured to mate with at least one of the plurality of perforations; and a piezoelectric actuator in communication with one or more of the first and second plates; wherein the capillary element transports the volatile composition from the reservoir to the first plate, and wherein the piezoelectric actuator, upon receiving an electrical signal, displaces at least one micro-plug from a seated position in at least one of the perforations and atomizes the volatile composition.

In yet another embodiment, there is provided, a method of atomizing a volatile composition, the method comprising the steps of: providing a volatile composition having a viscosity of greater than 5 cps, wherein the volatile composition is contained in a reservoir; providing an ultrasonic micro-plug unit comprising a first plate comprising a plurality of perforations extending through the first plate, a second plate comprising a plurality of micro-plugs extending longitudinally from base end to a tip end, and configured to mate with at least one of the plurality of perforations, a piezoelectric actuator in communication with one or more of the first and second plates; providing a capillary element for transporting the volatile composition from the reservoir to the first plate of the ultrasonic micro-plug unit; and actuating the piezoelectric actuator to atomize volatile composition from the micro-plug unit.

BRIEF DESCRIPTION OF THE DRAWINGS

In order to understand the invention and to see how it may be carried out in practice, embodiments will now be described, by way of non-limiting example only, with reference to the accompanying drawings, in which:

Figs. 1A-1H illustrate the micro-plug unit in different operational states which is not part of the present invention.
Fig. 2 illustrates a delivery system with a capillary element and reservoir for the improved volatile composition.

DETAILED DESCRIPTION OF EMBODIMENTS

The present invention provides a delivery system comprising improved volatile compositions and methods for atomizing such volatile compositions into the atmosphere by piezoelectric means.
It is to be understood that the delivery system is not limited to the construction and arrangement of the components set forth in the following description or illustrated in the drawings. The invention is applicable to other embodiments or of being practiced or carried out in various ways.

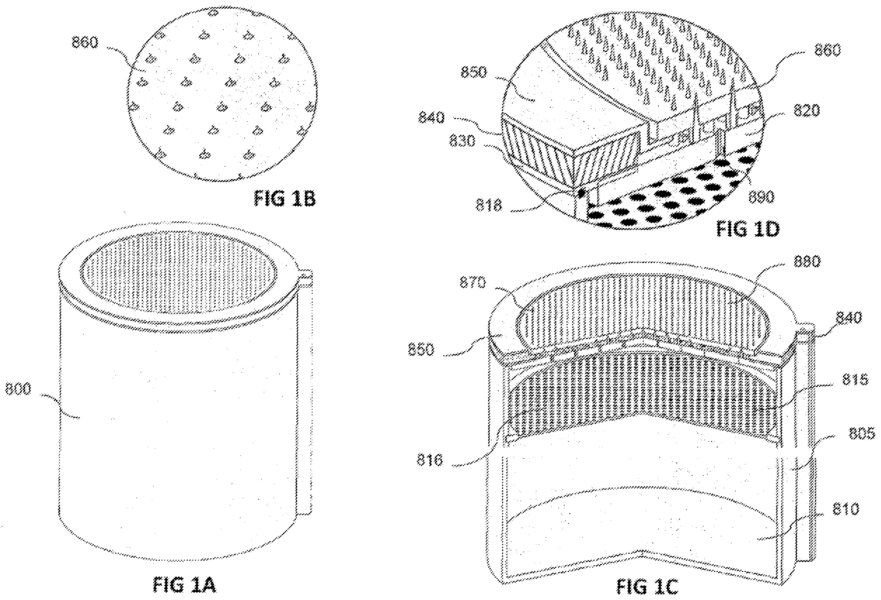

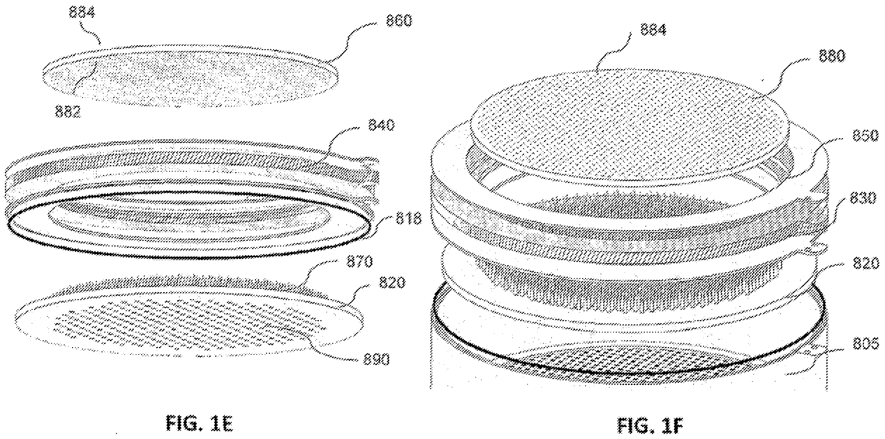

FIGS. 1A-1J illustrate various exploded views of an exemplary embodiment of a delivery system 800, which is not part of the present invention, the views being described taken together. Delivery system **800** may be used to atomize any volatile composition as defined in the claims. In particular, FIG. 1A illustrates an isometric view of delivery system **800;** FIG. 1B illustrates a more detailed view of a top portion of delivery system **800** of FIG. 1A;

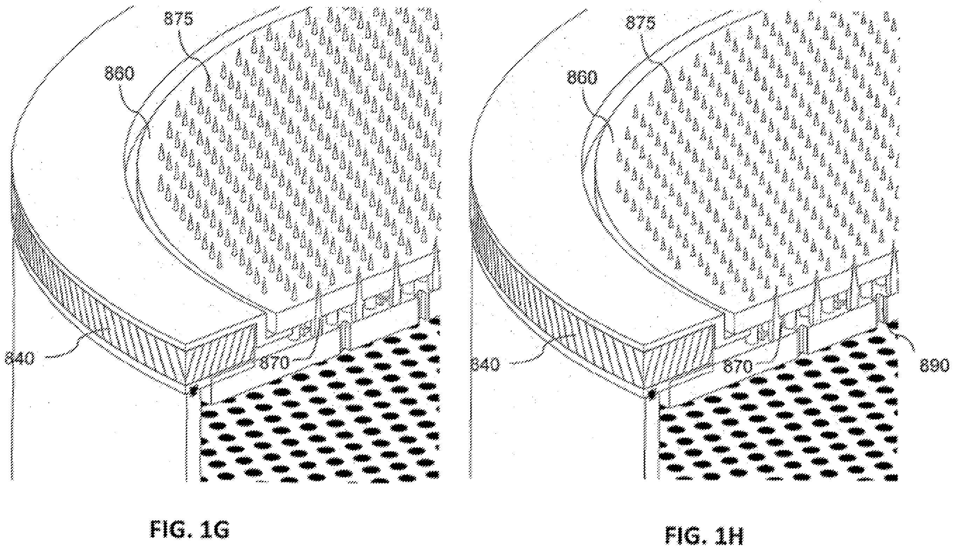

FIG. 1C illustrates an isometric cut away view of delivery system **800;** FIG. 1D illustrates a more detailed view of the top portion of FIG. 1B; FIG. IE illustrates a first exploded view of delivery system **800;** FIG. 1F illustrates a second exploded view of delivery system **800;** FIG. 1G illustrates a first position for certain portions of delivery system **800;** FIG. 1H illustrates a second position for certain portions of delivery system **800;** FIG. 1I illustrates a highly detailed view of the perforations and plugs of delivery system **800;** and FIG. 1J illustrates the resultant shape of scent producing liquid droplets, in accordance with an exemplary embodiment.
Delivery system **800** comprises: a housing **805;** a liquid reservoir **810** containing a volatile composition; a unidirectional flow membrane **815;** an holding tank**816;** a sealing ring **818;** a plug base **820;** a first electrode **830;** a piezoelectric actuator **840;** a second electrode **850;** and a plate **860.**

Ultrasonic micro-plug unit

The combination of the piezoelectric actuator **840,** micro-plugs **870,** and plate **860,** having perforations **880,** forms an ultrasonic micro-plug unit. Plate **860** exhibits a plurality of perforations **880** extending from first face **882** to second face **884.** Plug base **820** exhibits a plurality of pass through channels **890,** and a plurality of micro-plugs **870** extending longitudinally from a base portion attached to plug base **820** to a tip end **875,** with each plug arranged to mate with a respective one of perforations **880.** A portion of each plug **870,** and particularly the portion extending through perforations **880** may be conically shaped with an apex extending away from plug base **820.** Perforations **880** are preferably similarly conically shaped, such that when plug base **820** is brought to its closest position in relation to first face **882,** micro-plugs **870** are seated flush against the inner walls of the respective perforation **880.** The perforations **880** may exhibit a diameter of about 30 microns at first face **882,** matching the diameter of micro-plugs **870** when completely seated therein. The space created towards second face **884** when each micro-plug **870** is separated from the respective perforation **880** is denoted space and a ring shaped droplet of volatile scent liquid is formed by the shape of plug **870** in proximity of second face **884.** Sealing ring **818** is provided of a compliant material so as to form a seal against liquid, in particular a volatile scent liquid.
Piezoelectric actuator **840** is preferably a ring shaped element, and second electrode **850** is placed on the second side of piezoelectric actuator **840,** in electrical contact therewith. Plate **860** is placed in physical contact with second electrode **850,** and in one embodiment is secured thereto with an adhesive to prevent the escape of plate **860.** Face **882** of plate **860** is juxtaposed with plug base **820** such that each micro-plug **870** extends into a matching perforation **880.**

Reservoir and Flow Paths

Unidirectional flow membrane **815,** in cooperation with housing **805,** forms a liquid reservoir **810.** The delivery system **800** may be configured to have multiple reservoirs, each containing the same or a different composition. The reservoir **810** may also be formed as a separate construction so as to be replaceable. The reservoir **810** can be made of any suitable material for containing a liquid composition. Suitable materials for the containers include, but are not limited to, glass and plastic. Examples of such reservoirs are readily available in the marketplace.

According to the invention, the reservoir **810** comprises a capillary element **910** for dispensing the volatile material. The capillary element **910** may be any commercially available wicking material such as a fibrous or porous wick that contains multiple interconnected open cells which form capillary passages to draw a liquid composition up from the reservoir **810.** In one embodiment, the capillary element **910** may be a high density wick composition to aid in the containment of the scent or odor of a volatile composition. As used herein, high density wick compositions include any conventional wick material known in the art having a pore diameter ranging from about 20 microns to about 150 microns, alternatively from about 30 microns to about 70 microns, alternatively from about 30 microns to about 50 microns, alternatively, about 40 microns to about 50 microns. Non-limiting examples of suitable compositions include polyethylene, polypropylene, ethyl vinyl acetate, polyether sulfone, polyvinylidene fluoride, polytetrafluroethylene, polyethersulfone, and mixtures thereof.

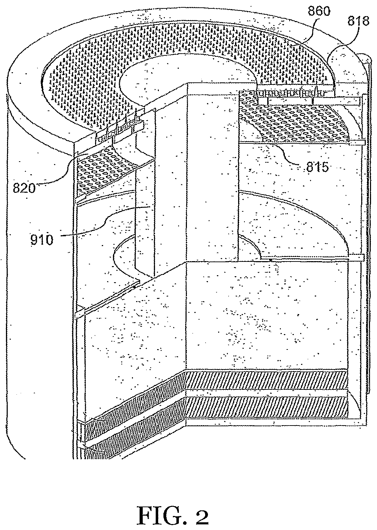

Referring to Fig. 2, the capillary element **910** may be at least partially in the reservoir **810.** In some embodiments, the capillary element **910** may be completely surrounded by the walls of the reservoir **810.** In one embodiment, the capillary element **910** is vertically aligned with the reservoir **810.** Depending upon the configuration of the delivery system **800,** a volatile composition may travel up or down the capillary element **910.** In the embodiment shown in Fig. 2, the volatile composition travels through the capillary element **910** in an upward motion.

After flowing through the capillary element **910,** the volatile composition may then continue traveling upstream to a holding tank **816.** Unidirectional flow membrane **815,** in cooperation with plug base **820,** defines the holding tank **816.** In particular, unidirectional flow membrane **815** defines the top of liquid reservoir **810** and the bottom of holding tank **816.** The top of holding tank **816** is defined by one end of plug base **820** in particular the side not exhibiting micro-plugs **870.** First electrode **830** is placed at one side of piezoelectric actuator **840,** in electrical contact therewith, and in physical contact with plug base **820,** in particular the side of plug base **820** exhibiting micro-plugs **870.**

In some embodiments, the delivery system **800** may include a fluid channel (not shown) positioned in a flow path between the capillary element **910** and the holding tank **816.** A channel may be useful in configurations where the reservoir **810** and holding tank **816** are placed laterally from one another. The length of the channel, measured from the capillary element **910** to center of the reservoir **810,** may be about 12 mm, alternatively about 13 mm, alternatively, about 14 mm, alternatively about 15 mm, alternatively about 11 mm, alternatively about 10 mm.

Operational Modes

In a first mode of operation, as illustrated in FIGS. 1G and 1H, a low frequency electric power is applied to piezoelectric actuator **840** to actuate the piezoelectric actuator 840. In response, the piezoelectric actuator **840** expands, separating plug base **820** from plate **860,** shown in FIG. 1G as being in closest proximity, to a separated position as illustrated in FIG. 1H. After expansion, an additional high frequency electrical power is further supplied, superimposed on the low frequency electrical power, vibrating piezoelectric actuator **840.** In an exemplary embodiment the high frequency electrical power exhibits a frequency range of 150-200 kHz, however this is not meant to be limiting in any way. The tapered shape of micro-plugs **870** function to focus the acoustical energy supplied by piezoelectric actuator **840** towards tip ends **875,** thereby atomizing any volatile scent liquid in contact with micro-plugs **870** and within perforations **880** so as to be scented distal of second face **884.** The tapered shape of micro-plugs **870** forms ring shaped droplet, which requires a reduced amount of energy to atomize when compared to a standard droplet of volatile liquid composition. The amount of separation between plug base **820** and plate **860** may be varied responsive to the viscosity of the composition being atomized, thus varying the dimensions of the ring shaped aperture produced by micro-plugs **870** in cooperation with perforations **880.** Thus, a single ultrasonic micro-plug unit may be utilized for liquids having a wide range of viscosity without being blocked.

In a second mode of operation, electrical power is disconnected from piezoelectric actuator **840,** and in response piezoelectric actuator **840** contracts bringing plug base **820** into closer proximity with plate **860** until micro-plugs **870** are seated flush within perforations **880,** i.e. a closed position, thus sealing the volatile liquid composition from second face **884,** and preventing any further scent from being experienced distal of second face **884.** The tapered shape mentioned above results in a complete seal, which is preferred for use with a volatile liquid composition; however this is not meant to be limiting in any way. In another embodiment, a complete seal is not required in the second mode, but only that the plurality of micro-plugs travel sufficiently through perforations **880** to ensure that no residual liquid remains within perforations **880** to prevent occlusion.

In a third mode of operation, while micro-plugs **870** are seated flush within perforations **880,** a medium to high frequency electrical power, in one non-limiting embodiment being from 40 kHz to 400 kHz, is supplied to piezoelectric actuator 840, thus vibrating the combination of plate **860** and micro-plugs **870.** Any residual volatile liquid composition on tip ends **875** and second face **884** is promptly atomized, or nebulized, and removed thus completely ceasing scent production.

In multiple reservoir delivery systems, a microprocessor and timer could be installed to emit the volatile composition from individual reservoirs at different times and for selected time periods, including emitting the volatile compositions in an alternating emission pattern as described in U.S. 7,223,361. Additionally, the delivery system could be programmable so a user can select certain compositions for emission. In the case of scented perfumes being emitted simultaneously, a customized scent may be delivered to the atmosphere.

Volatile composition

The volatile liquid composition of the present invention can encompass volatile materials including, but not limited, to volatile dyes, fragrance compositions, compositions that function as insecticides, air fresheners, deodorants, aromacology, aromatherapy, essential oils such as lavender, eucalyptus, or any other material that acts to condition, modify, or otherwise emit into the atmosphere or to modify the environment. Such materials may be included in the composition to assist with sleep, wake, respiratory health, and like conditions. Deodorants or malodor control compositions may comprise a material chosen from: odor neutralizing materials a non-limiting example of which is reactive aldehydes (as disclosed in U.S. 2005/0124512), odor blocking materials, odor masking materials, or sensory modifying materials, a non-limiting example of which is ionones (also disclosed in U.S. 2005/0124512), and combinations thereof.

In one embodiment, the volatile composition may comprise a perfume composition. When a perfume composition volatilizes into the air, the ingredients with the higher volatilities (referred to as "top notes") will be the ingredients that will volatilize and be detected by a person's sense of smell more quickly than the ingredients with lower volatilities (referred to as "middle notes") and the ingredients with the lowest volatility (referred to as "bottom notes"). This will cause the character of the perfume to change over time since after the perfume is first emitted, the overall perfume character will contain fewer and fewer top notes and more bottom notes.

The perfume compositions can include components that are suitably used in volatile composition emitting delivery systems such as the present invention. The components may be selected based on their Kovat's Index ("KI") (as determined on 5% phenyl-methylpolysiloxane as non-polar silicone stationary phase). The KI places the volatility attributes of an analyte (e.g. component of a volatile composition) on a gas chromatography column in relation to the volatility characteristics of an n-alkane (normal alkane) series on that column. A typical gas chromatograph ("GC") column is a DB-5 column available from Agilent Technologies of Palo Alto, California. By this definition, the KI of a normal alkane is set to 100n, where n is the number of carbon atoms in the n-alkane. The KI of an analyte, x, eluting at time t , between two n-alkanes with number of carbon atoms "n" and "N" having corrected retention times $t'_n$ and $t^t_N$ respectively, will then be calculated as:

$$ KI = 100 \left( n + \frac{\log t'_x - \log t'_n}{\log t'_N - \log t'_n} \right) $$

On a non-polar to slightly polar GC stationary phase, KI of analytes are correlated with their relative volatility. For example, analytes with smaller KIs tend to be more volatile than those with larger KIs. Ranking analytes with their corresponding KI values gives a good comparison of analyte evaporation rates in liquid-gas partitioning systems. The volatile composition according to the present invention can have at least one ingredient with a KI value of about 750 to about 2200, or about 800 to about 2200, or about 900 to about 2200, or about 1200 to about 2200, or about 1400 to about 2200, or about 1600 to about 2200.

In one embodiment, the volatile composition may comprise up to about 90%, by weight of the volatile composition, of perfume materials having a KI of 1200 to about 2200; alternatively from about 15% to about 50% by weight of the volatile composition, of perfume materials having a KI of 1400 to about 2200, alternatively from about 15% to about 30%, alternatively from about 15% to about 25%, alternatively from about 15% to about 20%; alternatively from about 15% to about 18%; alternatively from about 5% to about 20% by weight of the volatile composition, of perfume materials having a KI of 1600 to 2200, alternatively from about 5% to about 17%, alternatively from about 5% to about 15%, alternatively from about 5% to about 10%; alternatively from about 5% to about 8%; alternatively from about 0.2% to about 1%, by weight of the volatile composition, of perfume materials having a KI of greater than 1970 to about 2200, alternatively greater than 0.3% to about 0.5%.

A volatile composition may have a KI in any of the aforementioned KI ranges and have a vapor pressure from

8x10$^{-6}$ mm Hg to 5.50 mm Hg, measured at 25°C. In one embodiment, the volatile composition comprises a perfume material that is greater than 1400 KI and has a vapor pressure of less than 0.12 mm Hg.

Rather than, or in addition to KI, the perfume materials in the volatile composition can be selected based on their boiling point ("B.P.") and their octanol/water partition coefficient ("P"). The B.P. referred to herein is measured under normal standard pressure of 760 mm Hg. The B.P. of many perfume ingredients, at standard 760 mm Hg can be found in "Perfume and Flavor Chemicals (Aroma Chemicals)," written and published by Steffen Arctander, 1969.

The octanol/water partition coefficient of a perfume material is the ratio between its equilibrium concentrations in octanol and in water. The partition coefficients of the perfume ingredients used in the volatile composition may be more conveniently given in the form of their logarithm to the base 10, logP. The logP values of many perfume materials have been reported; see for example, the Pomona92 database, available from Daylight Chemical Information Systems, Inc. (Daylight CIS) of Irvine, California. However, the logP values are most conveniently calculated by the "CLOGP" program, also available from Daylight CIS. This program also lists experimental logP values when they are available in the Pomona92 database. The calculated logP ("ClogP") is determined by the fragment approach of Hansch and Leo (A. Leo, in Comprehensive Medicinal Chemistry, Vol. 4, C. Hansch, P. G. Sammens, J. B. Taylor and C. A. Ramsden, Eds., page 295, Pergamon Press, 1990). The fragment approach is based on the chemical structure of each perfume material, and takes into account the numbers and types of atoms, the atom connectivity, and chemical bonding. The ClogP values, which are the most reliable and widely used estimates for this physicochemical property, are typically used instead of the experimental logP values in the selection of perfume materials for the volatile composition of the present invention. In the present invention, the perfume materials may have a B.P. of greater than 250°C, alternatively about 250°C to about 400°C, alternatively about 260°C to about 375°C. The perfume raw materials may have the aforementioned B.P. and a ClogP value of less than 3, alternatively greater than 3, alternatively from about 1 to about 9. Such perfume raw materials may be present at any level in the volatile composition. In some embodiments, the volatile composition comprises greater than 15% to about 80%, by weight of the volatile composition, of perfume materials with a B.P. greater than 250°C, alternatively, greater than 15% to about 50%, alternatively greater than 15% to about 40%, alternatively greater than 15% to about 30%, alternatively greater than 15% to about 20%. Table 1 lists some non-limiting, exemplary materials having a B.P. greater than 250°C and a Clog P suitable for the present invention.

**Table 1**

| Perfume Material | B.P. (°C) | Clog P (at 25 °C) |
|---|---|---|
| iso E super | +250 | 3.455 |
| Eugenyl methyl ether | 251.5 | 2.673 |
| Acetaldehyde ethyl phenylethyl acetal | 253.2 | 2.351 |
| B enzyl-tert-butanol | 253.7 | 2.420 |
| Phenethyl isobutyrate | 254.7 | 2.967 |
| Anisyl acetate | 256.1 | 1.879 |
| Cinnamic alcohol | 256.1 | 1.408 |
| 6-Methylquinoline | 256.3 | 2.528 |
| Allyl phenoxyacetate | 257.2 | 2.253 |
| Frutene | 257.4 | 2.886 |
| Veratraldehyde | 257.6 | 1.240 |
| Lilial (p-t-bucinal) | 258 | 3.858 |
| Hydroxycitronellal dimethyl acetal | 259.3 | 1.640 |
| Dihydroeugenol | 259.7 | 2.881 |
| Cinnamyl acetate | 260.4 | 2.354 |
| Ethyl cinnamate | 261.1 | 2.994 |
| Amyl benzoate | 262 | 3.417 |
| Phenoxyethyl propionate | 262.7 | 2.614 |
| Eugenol | 263.3 | 2.397 |

(continued)

| Perfume Material | B.P. (°C) | Clog P (at 25 °C) |
|---|---|---|
| Heliotropin | 263.5 | 1.138 |
| Cinnamyl nitrile | 266.4 | 1.959 |
| Allyl cyclohexane propionate | 267 | 3.935 |
| exo-2-Camphanyl beta-hydroxyethyl ether | 267.3 | 2.597 |
| Ethyl 3-phenylglycidate | 267.5 | 2.195 |
| Coumarin | 268.5 | 1.412 |
| Scentenal | 269.6 | 0.924 |
| Anisylpropanal | 270.0 | 1.951 |
| Isoeugenol | 270.3 | 2.577 |
| Methyl lavender ketone | 270.7 | 2.413 |
| 2-Phenoxyethyl isobutyrate | 271.8 | 2.923 |
| Vanillin | 272.2 | 1.275 |
| Acetaldehyde phenylethyl propyl acetal | 274.6 | 2.880 |
| Jasmal | 275.7 | 2.379 |
| Ethyl methylphenylglycidate | 276.5 | 2.714 |
| Amyl cinnamic aldehyde | 285 | 4.324 |
| Ethyl vanillin | 286.1 | 1.804 |
| Isoeugenyl acetate | 286.6 | 2.283 |
| Heliotropine diethyl acetal | 288.3 | 2.062 |
| Cedrol | 291 | 4.530 |
| Hexadecanolide | 294 | 6.805 |
| Amyl cinnamic aldehyde dimethyl acetal | 300 | 4.033 |
| Ambrettolide | 300 | 6.261 |
| Phenyl ethyl benzoate | 300 | 4.058 |
| Dihydro isojasmonate | +300 | 3.009 |
| 2H-1,5-Benzodioxepin-3(4H)-one, 7-methyl- | 301.1 | 1.803 |
| 4-(4-Hydroxyphenyl)butanone-2 | 301.2 | 1.072 |
| Vanillin isobutyrate | 301.9 | 1.508 |
| Helional | 301.9 | 1.387 |
| Cashmeran | 302.4 | 2.373 |
| Cedryl acetate | 303 | 5.436 |
| Cyclohexyl salicylate | 304 | 5.265 |
| Hexyl cinnamic aldehyde | 305 | 5.473 |
| Benzophenone | 306 | 3.120 |
| Piperonyl acetone | 307.3 | 1.094 |
| Amyl cinnamate | 310 | 3.771 |
| Methyl beta-naphthyl ketone | 310.6 | 2.755 |
| Geranyl anthranilate | 312 | 4.216 |

(continued)

| Perfume Material | B.P. (°C) | Clog P (at 25 °C) |
|---|---|---|
| Methyl dihydrojasmonate | 314.3 | 2.419 |
| Lyral | 319.8 | 2.150 |
| Phenyl ethyl phenyl acetate | 325 | 3.767 |
| Ethylene brassylate | 332 | 4.554 |
| Cinnamyl cinnamate | 370 | 5.480 |
| Aurantiol | 450 | 4.216 |

When formulating volatile compositions, one may also include solvents, diluents, extenders, fixatives, thickeners, or the like. Non-limiting examples of these materials are ethyl alcohol, carbitol, diethylene glycol, dipropylene glycol, diethyl phthalate, triethyl citrate, isopropyl myristate, ethyl cellulose, and benzyl benzoate.

Table 2 shows one volatile composition comprising greater than 15%, by weight of the volatile composition, of perfume materials having a KI value greater than 1400.

**Table 2**

| Perfume Material | Approximate KI Value | % wt. | Approximate B.P. (°C) |
|---|---|---|---|
| Benzyl Acetate (CAS# 140-11-4) | 1173 | 1.5 | 214 |
| Ethyl-2-methyl Butyrate (CAS# 7452-79-1) | 850 | 0.3 | 132 |
| Amyl Acetate (CAS# 628-63-7) | 912 | 1.0 | 149 |
| Cis 3 Hexenyl Acetate (CAS# 3681-71-8) | 1009 | 0.5 | 166 |
| Ligustral (CAS# 27939-60-2) | 1094 | 0.5 | 177 |
| Melonal (CAS# 106-72-9) | 1060 | 0.5 | 116 |
| Hexyl Acetate (CAS# 142-92-7) | 1016 | 2.5 | 146 |
| Benzyl Salicylate (CAS# 118-58-1) | 2139 | 3 | 320 |
| Coumarin (CAS# 91-64-5) | 1463 | 1.5 | 267 |
| Methyl Dihydro Jasmonate (CAS# 24851-98-7) | 1668 | 7 | 314 |
| Hexyl Cinnamic Aldehyde (CAS# 101-86-0) | 1770 | 6 | 305 |
| Dipropylene Glycol Methyl Ether (CAS# 34590-94-8) | 997 | 75.7 | 190 |

In some embodiments, the volatile composition may contain functional perfume components ("FPCs"). FPCs are a class of perfume raw materials with evaporation properties that are similar to traditional organic solvents or volatile organic compounds ("VOCs"). "VOCs", as used herein, means volatile organic compounds that have a vapor pressure of greater than 0.2 mm Hg measured at 20°C and aid in perfume evaporation. Exemplary VOCs include the following organic solvents: dipropylene glycol methyl ether ("DPM"), 3-methoxy-3-methyl-1-butanol ("MMB"), volatile silicone oil, and dipropylene glycol esters of methyl, ethyl, propyl, butyl, ethylene glycol methyl ether, ethylene glycol ethyl ether, diethylene glycol methyl ether, diethylene glycol ethyl ether, or any VOC under the tradename of Dowanol™ glycol ether. VOCs are commonly used at levels greater than 20% in a liquid composition to aid in perfume evaporation.

The FPCs of the present invention aid in the evaporation of perfume materials and may provide a hedonic, fragrance benefit. FPCs may be used in relatively large concentrations without negatively impacting perfume character of the overall composition. As such, in some embodiments, the volatile composition of the present invention may be substantially free of VOCs, meaning it has no more than 18%, alternatively no more than 6%, alternatively no more than 5%, alternatively no more than 1%, alternatively no more than 0.5%, by weight of the composition, of VOCs. The volatile composition, in some embodiments, may be free of VOCs.

Perfume materials that are suitable as a FPC may have a KI, as defined above, from about 800 to about 1500, alternatively about 900 to about 1200, alternatively about 1000 to about 1100, alternatively about 1000.

Perfume materials that are suitable for use as a FPC can also be defined using odor detection threshold ("ODT") and non-polarizing scent character for a given perfume character scent camp. ODTs may be determined using a commercial GC equipped with flame ionization and a sniff-port. The GC is calibrated to determine the exact volume of material injected by the syringe, the precise split ratio, and the hydrocarbon response using a hydrocarbon standard of known concentration and chain-length distribution. The air flow rate is accurately measured and, assuming the duration of a human inhalation to last 12 seconds, the sampled volume is calculated. Since the precise concentration at the detector at any point in time is known, the mass per volume inhaled is known and concentration of the material can be calculated. To determine whether a material has a threshold below 50 ppb, solutions are delivered to the sniff port at the back-calculated concentration. A panelist sniffs the GC effluent and identifies the retention time when odor is noticed. The average across all panelists determines the threshold of noticeability. The necessary amount of analyte is injected onto the column to achieve a 50 ppb concentration at the detector. Typical GC parameters for determining ODTs are listed below. The test is conducted according to the guidelines associated with the equipment.

Equipment:

GC: 5890 Series with FID detector (Agilent Technologies, Ind., Palo Alto, California, USA);
7673 Autosampler (Agilent Technologies, Ind., Palo Alto, California, USA);
Column: DB-1 (Agilent Technologies, Ind., Palo Alto, California, USA) Length 30 meters ID 0.25 mm film thickness 1 micron (a polymer layer on the inner wall of the capillary tubing, which provide selective partitioning for separations to occur). Method Parameters:
Split Injection: 17/1 split ratio;
Autosampler: 1.13 microliters per injection;
Column Flow: 1.10 mL/minute;
Air Flow: 345 mL/minute;
Inlet Temp. 245°C;
Detector Temp. 285°C.

Temperature Information:

Initial Temperature: 50°C;
Rate: 5C/minute;
Final Temperature: 280°C;
Final Time: 6 minutes;
Leading assumptions:

(i) 12 seconds per sniff
(ii) GC air adds to sample dilution.

FPCs may have an ODT from greater than about 1.0 parts per billion ("ppb"), alternatively greater than about 5.0 ppb, alternatively greater than about 10.0 ppb, alternatively greater than about 20.0 ppb, alternatively greater than about 30.0 ppb, alternatively greater than about 0.1 parts per million.

For energized air fresheners, as a non-limiting example, the FPCs may have a KI in the range from about 900 to about 1400; alternatively from about 1000 to about 1300. These FPCs can be either an ether, an alcohol, an aldehyde, an acetate, a ketone, or mixtures thereof.

In addition to KI and ODT properties mentioned above, other physical chemical properties of perfume raw materials that may render them useful as a FPC are molecular weight, vapor pressure, B.P., flashpoint, heat of vaporization, viscosity, solubility parameters, and combinations thereof.

FPCs may be highly volatile, low B.P. perfume materials. Exemplary FPC include iso-nonyl acetate, dihydro myrcenol (3-methylene-7-methyl octan-7-ol), linalool (3-hydroxy-3, 7-dimethyl-1, 6 octadiene), geraniol (3, 7 dimethyl-2, 6-octadien-1-ol), d-limonene (1-methyl-4-isopropenyl-1-cyclohexene, benzyl acetate, isopropyl mystristate, and mixtures thereof. Table 3 lists the approximate reported values for exemplary properties of certain FPCs.

**Table 3**

| FPC | B.P. (°C) | MW | Clog P @ 25°C | Flash point (°C) | Vapor pressure | KI | ODT |
|---|---|---|---|---|---|---|---|
| Iso-Nonyl Acetate (CAS# 58430-94-7) | 224.72 | 186.3 | 4.28 | 79.4 | 0.11 | 1178 | 12ppb |
| Dihydro Myrcenol (CAS# 18479-58-8) | 197.66 | 156.3 | 3.03 | 76.1 | 0.1 | 1071 | 32ppb |
| Linalool (CAS# 78-70-6) | 205.1 | 154.3 | 2.549 | 78.9 | 0.05 | 1107 | 22ppb |
| Geraniol (CAS# 106-24-1) | 237.4 | 154.3 | 2.769 | 100 | 0.00519 | 1253 | 0.4ppb |
| D-Limonene (CAS# 94266-47-4) | 169.7 | 136 | 4.35 | 47.2 | 1.86 | 1034 | 204ppb |

The total amount of FPCs in the volatile composition of the present invention may be greater than about 50%, alternatively greater than about 60%, alternatively greater than about 70%, alternatively greater than about 75%, alternatively greater than about 80%, alternatively from about 50% to about 100%, alternatively from about 60% to about 100%, alternatively from about 70% to about 100%, alternatively from about 75% to about 100%, alternatively from about 80% to about 100%, alternatively from about 85% to about 100%, alternatively from about 90% to about 100%, alternatively about 100%, by weight of the perfume mixture. In some embodiments, the volatile composition may consist entirely of FPCs (i.e. 100 wt. %).

For purposes of illustrating the present invention in further detail, Table 4 lists a non-limiting, exemplary volatile composition comprising FPCs and their approximate reported values for KI and B.P.

**Table 4**

| Material Name | KI | % w/w | B.P. (°C) |
|---|---|---|---|
| Benzyl Acetate (CAS # 140-11-4) | 1173 | 1.5 | 214 |
| Ethyl-2-methyl Butyrate (CAS # 7452-79-1) | 850 | 0.3 | 132 |
| Amyl Acetate (CAS # 628-63-7) | 912 | 1.0 | 149 |
| Cis 3 Hexenyl Acetate (CAS # 3681-71-8) | 1009 | 0.5 | 166 |
| Ligustral (CAS # 27939-60-2) | 1094 | 0.5 | 177 |
| Melonal (CAS # 106-72-9) | 1060 | 0.5 | 116 |
| Hexyl Acetate (CAS # 142-92-7) | 1016 | 2.5 | 146 |
| Dihydro Myrcenol (CAS# 18479-58-8) | 1071 | 15 | 198 |
| Phenyl Ethyl Alcohol (CAS# 60-12-8) | 1122 | 8 | 219 |
| Linalool (CAS # 78-70-6) | 1243 | 25.2 | 205 |
| Geraniol (CAS# 106-24-1) | 1253 | 5 | 237 |
| Iso Nonyl Acetate (CAS# 40379-24-6) | 1295 | 22.5 | 225 |
| Benzyl Salicylate (CAS # 118-58-1) | 2139 | 3 | 320 |
| Coumarin (CAS # 91-64-5) | 1463 | 1.5 | 267 |
| Methyl Dihydro Jasmonate (CAS# 24851-98-7) | 1668 | 7 | 314 |
| Hexyl Cinnamic Aldehyde (CAS # 101-86-0) | 1770 | 6 | 305 |

The volatile composition of the present invention may also exhibit a viscosity greater than 5 centipoise ("cps"), alternatively greater than or equal to 10 cps. And, the volatile composition may have surface tensions below about 35, alternatively in the range of from about 20 to about 30 dynes per centimeter. Viscosity is in cps, as determined using the Bohlin CVO Rheometer system in conjunction with a high sensitivity double gap geometry. Surface Tension results, in dynes per centimeter, were generated using the Kruss K-12 tensionmeter operating under the Wilhelmy Plate protocol.

Other Optional Features

Heat/Fan

In a non-limiting embodiment, a small amount of heat could be used to improve flow rate of the volatile composition from the ultrasonic micro-plug unit and thus improve room-fill. The delivery system may include a heating element positioned adjacent the ultrasonic micro-plug unit and/or the reservoir. Heat from the heating element can be generated by known means in the art. For example, resistors may be secured to an element of the ultrasonic micro-plug unit using any commercially available heat conductive adhesive. One heat conductive adhesive is TRA-BOND 2151 two-part, epoxy made by TRA-CON. The heat will be distributed by means of thermal conduction. The temperature of the heating element may be controlled by thermistor, which is a type of resistor with resistance varying according to its temperature, used in conjunction with a microcontroller. Effective temperatures are from about 18°C to about 29°C.

In another aspect of the invention, the delivery system may comprise a fan to assist in driving room-fill. The fan may be any known fan used in the art for air freshening systems.

Sensors

In some embodiments, the delivery system may include commercially available sensors that respond to environmental stimuli such as light, noise, motion, and/or odor levels in the air. For example, the delivery system can be programmed to turn on when it senses light, and/or to turn off when it senses no light. In another example, the delivery system can turn on when the sensor senses a person moving into the vicinity of the sensor. Sensors may also be used to monitor the odor levels in the air. The odor sensor can be used to turn-on the delivery system, increase the heat or fan speed, and/or step-up the atomization of droplets from the ultrasonic micro-plug unit when it is needed.

The sensor may also be used to measure fluid levels in the reservoir to indicate the reservoir's "end of life" in advance of depletion. In such case, a light may turn on to indicate the reservoir needs to be replaced or filled.

The sensors may be integral with the delivery system housing or in a remote location (i.e. physically separated from the delivery system housing).

Portable / Battery

The delivery system may be configured to compact and easily portable. In such case, the delivery system may be battery operated. The delivery system may be capable for use with electrical sources as 9-volt batteries, conventional dry cells such as "A", "AA", "AAA", "C", and "D" cells, button cells, watch batteries, solar cells, as well as rechargeable batteries with recharging base.

Programming

The delivery system may include programmable electronics to set a precise delivery rate (in milligrams per hour). Alternatively, the electronic circuitry may allow the consumer to adjust intensity or effectiveness to a desired level for personal preference, efficacy, or for room size.

Throughout this specification, components referred to in the singular are to be understood as referring to both a single or plural of such component.

All percentages stated herein are by weight unless otherwise specified.

Every numerical range given throughout this specification will include every narrower numerical range that falls within such broader numerical range, as if such narrower numerical range were all expressly written herein. For example, a stated range of "1 to 10" should be considered to include any and all subranges between (and inclusive of) the minimum value of 1 and the maximum value of 10; that is, all subranges beginning with a minimum value of 1 or more and ending with a maximum value of 10 or less, e.g., 1 to 6.1, 3.5 to 7.8, 5.5 to 10, etc.

Further, the dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document cited, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

**Claims**

1. A delivery system (800) comprising:

   a volatile composition comprising from 15% to 50%, by weight of said volatile composition, of materials having a Kovat's Index greater than 1400, wherein said volatile composition is contained in a reservoir (810) having a capillary element (910);
   an ultrasonic micro-plug unit comprising

   a first plate (860) comprising a plurality of perforations (880) extending through said first plate (860);
   a second plate (820) comprising a plurality of micro-plugs (870) extending longitudinally from base end to a tip end, and configured to mate with at least one of said plurality of perforations (880); and
   a piezoelectric actuator (840) in communication with one or more of said

   first and second plates (860, 820);

   wherein said capillary element (910) transports said volatile composition from said reservoir (810) to said first plate (860), and wherein said piezoelectric actuator (840), upon receiving an electrical signal, displaces at least one micro-plug from a seated position in at least one of said perforations (880) and atomizes said volatile composition.

2. The delivery system of Claim 1, wherein said volatile composition comprises 15% to 20%, by weight of said volatile composition, of materials having a Kovat's Index greater than 1400, preferably 1400 to 2200.

3. The delivery system of Claim 1, wherein said volatile composition comprises from 5% to 20%, by weight of said volatile composition, of materials having a Kovat's Index greater than 1600, preferably 0.3% to 0.5%, by weight of said volatile composition, of materials having a Kovat's Index of 1970 to 2200.

4. The delivery system of Claim 1, wherein said volatile composition comprises a viscosity of greater than 5 cps.

5. The delivery system of Claim 1, wherein said materials comprise a Kovat's Index greater than 1400 and a vapor pressure of less than 0.12mm Hg at 25°C.

6. The delivery system of Claim 1 further comprising a sensor selected from the group consisting of a motion sensor, a light sensor, a fluid detection sensor, an odor detection sensor, and combinations thereof.

7. A delivery system (800) comprising:

   a volatile composition comprising greater than 50%, by weight of said volatile composition, of at least one FPC, wherein said volatile composition is contained in a reservoir (810) having a capillary element (910);
   an ultrasonic micro-plug unit comprising

   a first plate (810) comprising a plurality of perforations (880) extending through said first plate (860);
   a second plate (820) comprising a plurality of micro-plugs (870) extending longitudinally from base end to a tip end, and configured to mate with at least one of said plurality of perforations (880); and
   a piezoelectric actuator (840) in communication with one or more of said

   first and second plates (860, 820);

   wherein said capillary element (910) transports said volatile composition from said reservoir (810) to said first plate (860), and wherein said piezoelectric actuator (840), upon receiving an electrical signal, displaces at least one micro-plug from a seated position in at least one of said perforations (880) and atomizes said volatile composition.

8. The delivery system of Claim 7, wherein the at least one FPC is present in said volatile composition in an amount greater than 75%, by weight of said volatile composition, preferably greater than 85%, by weight of said volatile composition.

9. The delivery system of Claim 7, wherein the at least one FPC is selected from the group consisting of: iso-nonyl acetate, dihydro myrcenol (3-methylene-7-methyl octan-7-ol), linalool (3-hydroxy-3, 7-dimethyl-1, 6 octadiene), geraniol (3, 7 dimethyl-2, 6-octadien-1-ol), d-limonene (1-methyl-4-isopropenyl-1-cyclohexene, benzyl acetate, isopropyl mystristate, and mixtures thereof.

10. The delivery system of Claim 7, wherein the volatile composition comprises a mixture of FPCs comprising iso-nonyl acetate, dihydro myrcenol (3-methylene-7-methyl octan-7-ol), linalool (3-hydroxy-3, 7-dimethyl-1, 6 octadiene), and geraniol (3, 7 dimethyl-2, 6-octadien-1-ol).

11. A delivery system (800) comprising:

a volatile composition comprising greater than 15%, by weight of said volatile composition, of materials having a boiling point greater than 250°C, wherein said volatile composition is contained in a reservoir (810) having a capillary element (910);
an ultrasonic micro-plug unit comprising

a first plate (860) comprising a plurality of perforations (880) extending through said first plate (860);
a second plate (820) comprising a plurality of micro-plugs (870) extending longitudinally from base end to a tip end, and configured to mate with at least one of said plurality of perforations (880); and
a piezoelectric actuator (840) in communication with one or more of said

first and second plates (860, 820);

wherein said capillary element (910) transports said volatile composition from said reservoir (810) to said first plate (860), and wherein said piezoelectric actuator (840), upon receiving an electrical signal, displaces at least one micro-plug from a seated position in at least one of said perforations (880) and atomizes said volatile composition.

12. A method of atomizing a volatile composition, said method comprising the steps of:

providing a volatile composition having a viscosity of greater than 5 cps, wherein said volatile composition is contained in a reservoir (810);
providing an ultrasonic micro-plug unit comprising

a first plate (860) comprising a plurality of perforations (880) extending through said first plate (860),
a second plate (820) comprising a plurality of micro-plugs (870) extending longitudinally from base end to a tip end, and configured to mate with at least one of said plurality of perforations (880),
a piezoelectric actuator (840) in communication with one or more of said first and second plates (860, 820);

providing a capillary element (910) for transporting said volatile composition from said reservoir (810) to said first plate (860) of said ultrasonic micro-plug unit; and
actuating said piezoelectric actuator (840) to atomize volatile composition from said micro-plug unit.

13. The method of claim 12, wherein electrical power is delivered from a power source to said piezoelectric actuator in a manner to provide intermittent production of said droplets.

14. The method of claim 12, wherein said volatile composition has a viscosity of greater than 10 centipoise, preferably greater than 11 centipoise.

15. The method of claim 12 further comprising providing a diffusion assistance element to aid in atomizing said volatile composition, said diffusion assistance element is selected from: a fan, a heating element, and combinations thereof.

**Patentansprüche**

1. Abgabesystem (800), umfassend:

   eine flüchtige Zusammensetzung, die 15 Gew.-% bis 50 Gew.-% der flüchtigen Zusammensetzung aus Materialien umfasst, die einen Kovat's Index von mehr als 1400 haben, wobei die flüchtige Zusammensetzung in einem Behälter (810) enthalten ist, der ein Kapillarelement (910) aufweist;
   eine Ultraschall-Mikrosteckereinheit, umfassend
   eine erste Platte (860), die mehrere Perforationen (880) umfasst, die sich durch die erste Platte erstrecken (860);
   eine zweite Platte (820), die mehrere Mikrostecker (870) umfasst, die sich in Längsachse vom unteren Ende zu einem Spitzenende erstreckt, und konfiguriert ist, um mit mindestens einem der mehreren Perforationen (880) zusammenzupassen; und
   einen piezoelektrischen Aktor (840), der eine oder mehrere der ersten und zweiten Platten (860, 820) berührt; wobei das Kapillarelement (910) die flüchtige Zusammensetzung aus dem Behälter (810) zur ersten Platte (860) transportiert, und wobei der piezoelektrische Aktor (840) auf den Empfang eines elektrischen Signals hin mindestens einen Mikrostecker von einer sitzenden Position in mindestens eine der Perforationen (880) verschiebt und die flüchtige Zusammensetzung atomisiert.

2. Abgabesystem nach Anspruch 1, wobei die flüchtige Zusammensetzung 15 Gew.-% bis 20 Gew.-% der flüchtigen Zusammensetzung an Materialien umfasst, die einen Kovat-Index von mehr als 1400 aufweisen, bevorzugt von mehr als 1400 bis 2200.

3. Abgabesystem nach Anspruch 1, wobei die flüchtige Zusammensetzung 5 Gew.-% bis 20 Gew.-% der flüchtigen Zusammensetzung an Materialien umfasst, die einen Kovat-Index von mehr als 1600 aufweisen, bevorzugt 0,3 Gew.-% bis 0,5 Gew.-% der flüchtigen Zusammensetzung an Materialien, die einen Kovat-Index von 1970 bis 2200 aufweisen.

4. Abgabesystem nach Anspruch 1, wobei die flüchtige Zusammensetzung eine Viskosität von mehr als 5 cps aufweist.

5. Abgabesystem nach Anspruch 1, wobei die Materialien einen Kovat-Index von mehr als 1400 und einen Dampfdruck von weniger als 0,02 kPa (0,12 mm Hg) bei 25 °C aufweisen.

6. Abgabesystem nach Anspruch 1, ferner umfassend einen Sensor, der ausgewählt ist aus der Gruppe, bestehend aus einem Bewegungssensor, einem Lichtsensor, einem Fluiddetektionssensor, einen Geruchsdetektionssensor, und Kombinationen davon.

7. Abgabesystem (800), umfassend:

   eine flüchtige Zusammensetzung, die mehr als 50 Gew.-% der flüchtigen Zusammensetzung von mindestens einem FPC umfasst, wobei die flüchtige Zusammensetzung in einem Behälter (810) enthalten ist, der ein Kapillarelement (910) aufweist.
   eine Ultraschall-Mikrosteckereinheit, umfassend
   eine erste Platte (810), die mehrere Perforationen (880) umfasst, die sich durch die erste Platte (860) erstrecken;
   eine zweite Platte (820), die mehrere Mikrostecker (870) umfassen, die sich in Längsrichtung von dem unteren Ende zum Spitzenende erstrecken, und angepasst sind, um mit wenigstens einem der mehreren Perforationen (880) zusammenzupassen; und
   einen piezoelektrischen Aktor (840), der eine oder mehrere der ersten und zweiten Platten (860, 820) berührt; wobei das Kapillarelement (910) die flüchtige Zusammensetzung aus dem Behälter (810) zur ersten Platte (860) transportiert, und wobei der piezoelektrische Aktor (840) auf den Empfang eines elektrischen Signals hin mindestens einen Mikrostecker von einer sitzenden Position in mindestens eine der Perforationen (880) verschiebt und die flüchtige Zusammensetzung atomisiert.

8. Abgabesystem nach Anspruch 7, wobei das mindestens eine FPC in der flüchtigen Zusammensetzung in einer Menge vorliegt, die größer als 75 Gew.-% der flüchtigen Zusammensetzung ist, bevorzugt größer als 85 Gew.-% der flüchtigen Zusammensetzung.

9. Abgabesystem nach Anspruch 7, wobei das wenigstens eine FPC ausgewählt ist aus der Gruppe, bestehend aus: Iso-nonylacetat, Dihydromyrcenol(3-methylen-7-methyl octan-7-ol), Linalool (3-hydroxy-3, 7-dimethyl-1,6 octadien), Geraniol (3,7 dimethyl-2,6-octadien-1-ol), d-Limonen(1-methyl-4-isopropenyl-1-cyclohexen, Benzylacetat, Isopropylmystristat, und Mischungen davon.

10. Abgabesystem nach Anspruch 7, wobei die flüchtige Zusammensetzung eine Mischung von FPCs umfasst, welche Iso-nonylacetat, Dihydromyrcenol(3-methylen-7-methyl octan-7-ol), Linalool (3-hydroxy-3, 7-dimethyl-1,6 octadien), und Geraniol (3,7 dimethyl-2,6-octadien-1-ol) umfasst.

11. Abgabesystem (800), umfassend:

eine flüchtige Zusammensetzung, die mehr als 15 Gew.-% der flüchtigen Zusammensetzung an Materialien umfasst, deren Siedepunkt größer als 250 °C ist, wobei die flüssige Zusammensetzung in einem Behälter (810) enthalten ist, der ein Kapillarelement (910) aufweist;
eine Ultraschall-Mikrosteckereinheit, umfassend
eine erste Platte (860), die mehrere Perforationen (880) umfasst, die sich durch die erste Platte erstrecken (860);
eine zweite Platte (820), die mehrere Mikrostecker (870) umfassen, die sich in Längsrichtung von dem unteren Ende zum Spitzenende erstrecken, und angepasst sind, um mit wenigstens einem der mehreren Perforationen (880) zusammenzupassen; und
einen piezoelektrischen Aktor (840), der eine oder mehrere der ersten und zweiten Platten (860, 820) berührt;
wobei das Kapillarelement (910) die flüchtige Zusammensetzung aus dem Behälter (810) zur ersten Platte (860) transportiert, und wobei der piezoelektrische Aktor (840) auf den Empfang eines elektrischen Signals hin mindestens einen Mikrostecker von einer sitzenden Position in mindestens eine der Perforationen (880) verschiebt und die flüchtige Zusammensetzung atomisiert.

12. Verfahren zur Atomisierung einer flüchtigen Zusammensetzung, wobei das Verfahren die folgenden Schritte umfasst:

zur-Verfügung-Stellung einer flüchtigen Zusammensetzung mit einer Viskosität von mehr als 5 cps, wobei die flüchtige Zusammensetzung in einem Behälter (810) enthalten ist.
Bereitstellung einer Ultraschall-Mikrostecker-Einheit, umfassend
eine erste Platte (860), die mehrere Perforationen (880) umfasst, die sich durch die erste Platte (860) erstrecken;
eine zweite Platte (820), die mehrere Mikrostecker (870) umfassen, die sich in Längsrichtung von dem unteren Ende zum Spitzenende erstrecken, und angepasst sind, um mit wenigstens einem der mehreren Perforationen (880) zusammenzupassen;
einen piezoelektrischen Aktor (840), der eine oder mehrere der ersten und zweiten Platten (860, 820) berührt;
Bereitstellung eines Kapillarelementes (910) zum Transport der flüssigen Zusammensetzung von dem Behälter (810) zur ersten Platte (860) der Ultraschall-Mikrosteckereinheit; und
Betätigen des piezoelektrischen Aktors (840) zur Atomisierung der flüchtigen Zusammensetzung von der Mikrosteckereinheit.

13. Verfahren nach Anspruch 12, wobei elektrischer Strom von einer Stromquelle zu einem piezolektrischen Aktor in einer Art und Weise übertragen wird, dass eine intermittierende Erzeugung der Tröpfchen bereitgestellt wird.

14. Verfahren nach Anspruch 12, wobei die flüchtige Zusammensetzung eine Viskosität von mehr als 10 Centipoise, bevorzugt mehr als 11 Centipoise, aufweist.

15. Verfahren nach Anspruch 12, ferner umfassend das Bereitstellen eines Diffusionsunterstützungselementes zur Unterstützung der Atomisierung der flüchtigen Zusammensetzung, wobei das Diffusionsunterstützungselement ausgewählt ist aus:

einem Ventilator, einem Heizelement, und Kombinationen davon.

## Revendications

1. Système de distribution (800) comprenant :

une composition volatile comprenant de 15 % à 50 % en poids de ladite composition volatile, de matériaux possédant un indice de Kovat supérieur à 1400, dans lequel ladite composition volatile est contenue dans un réservoir (810) comportant un élément capillaire (910) ;

une unité ultrasonore de micro-bouchons comprenant

une première plaque (860) comprenant une pluralité de perforations (880) s'étendant à travers ladite première plaque (860) ;

une deuxième plaque (820) comprenant une pluralité de micro-bouchons (870) s'étendant longitudinalement de l'extrémité de base à une extrémité de bout, et conçue pour s'accoupler avec au moins une parmi ladite pluralité de perforations (880) ; et

un actionneur piézoélectrique (840) en communication avec une ou plusieurs desdites première et deuxième plaques (860, 820) ;

dans lequel ledit élément capillaire (910) transporte ladite composition volatile dudit réservoir (810) à ladite première plaque (860), et dans lequel ledit actionneur piézoélectrique (840), lors de la réception d'un signal électrique, déplace au moins un micro-bouchon d'une position en place dans au moins une desdites perforations (880) et atomise ladite composition volatile.

2. Système de distribution selon la revendication 1, dans lequel ladite composition volatile comprend 15 % à 20 % en poids de ladite composition volatile, de matériaux possédant un indice de Kovat supérieur à 1400, de préférence 1400 à 2200.

3. Système de distribution selon la revendication 1, dans lequel ladite composition volatile comprend de 5 % à 20 % en poids de ladite composition volatile, de matériaux ayant un indice de Kovat supérieur à 1600, de préférence 0,3 % à 0,5 % en poids de ladite composition volatile, de matériaux ayant un indice de Kovat de 1970 à 2200.

4. Système de distribution selon la revendication 1, dans lequel ladite composition volatile comprend une viscosité supérieure à 5 cP.

5. Système de distribution selon la revendication 1, dans lequel lesdits matériaux comprennent un indice de Kovat supérieur à 1400 et une pression de vapeur inférieure à 0,02 kPa (0,12 mm Hg) à 25 °C.

6. Système de distribution selon la revendication 1 comprenant en outre un capteur choisi dans le groupe constitué d'un capteur de mouvement, un capteur de lumière, un capteur de détection de fluide, un capteur de détection d'odeur, et des combinaisons de ceux-ci.

7. Système de distribution (800) comprenant :

une composition volatile comprenant plus de 50 % en poids de ladite composition volatile, d'au moins un composant de parfum fonctionnel (CPF), dans lequel ladite composition volatile est contenue dans un réservoir (810) comportant un élément capillaire (910) ;

une unité ultrasonore de micro-bouchons comprenant

une première plaque (810) comprenant une pluralité de perforations (880) s'étendant à travers ladite première plaque (860) ;

une deuxième plaque (820) comprenant une pluralité de micro-bouchons (870) s'étendant longitudinalement de l'extrémité de base à une extrémité de bout, et conçue pour s'accoupler avec au moins une parmi ladite pluralité de perforations (880) ; et

un actionneur piézoélectrique (840) en communication avec une ou plusieurs desdites première et deuxième plaques (860, 820) ;

dans lequel ledit élément capillaire (910) transporte ladite composition volatile dudit réservoir (810) à ladite première plaque (860), et dans lequel ledit actionneur piézoélectrique (840), lors de la réception d'un signal électrique, déplace au moins un micro-bouchon d'une position en place dans au moins une desdites perforations (880) et atomise ladite composition volatile.

8. Système de distribution selon la revendication 7, dans lequel l'au moins un CPF est présent dans ladite composition volatile en une quantité supérieure à 75 % en poids de ladite composition volatile, de préférence supérieure à 85 % en poids de ladite composition volatile.

9. Système de distribution selon la revendication 7, dans lequel l'au moins un CPF est choisi dans le groupe constitué de : acétate d'iso-nonyle, dihydromyrcénol (3-méthylène-7-méthyl-octan-7-ol), linalol-(3-hydroxy-3,7-diméthyl-1,6 octadiène), géraniol-(3,7-diméthyl-2,6-octadién-1-ol), d-limonène-(1-méthyl-4-isopropényl-1-cyclohexène, acétate de benzyle, mystristate d'isopropyle, et des mélanges de ceux-ci.

**10.** Système de distribution selon la revendication 7, dans lequel la composition volatile comprend un mélange de CPF comprenant de l'acétate d'iso-nonyle, du dihydro-myrcénol-(3-méthylène-7-méthyl-octan-7-ol), du linalol-(3-hydroxy-3,7-diméthyl-1,6-octadiène), et du géraniol-(3,7-diméthyl-2,6-octadién-1-ol).

**11.** Système de distribution (800) comprenant :

une composition volatile comprenant plus de 15 % en poids de ladite composition volatile, de matériaux possédant un point d'ébullition supérieur à 250 °C, dans lequel ladite composition volatile est contenue dans un réservoir (810) ayant un élément capillaire (910) ;
une unité ultrasonore de micro-bouchons comprenant
une première plaque (860) comprenant une pluralité de perforations (880) s'étendant à travers ladite première plaque (860) ;
une deuxième plaque (820) comprenant une pluralité de micro-bouchons (870) s'étendant longitudinalement de l'extrémité de base à une extrémité de bout, et conçue pour s'accoupler avec au moins une parmi ladite pluralité de perforations (880) ; et
un actionneur piézoélectrique (840) en communication avec une ou plusieurs desdites première et deuxième plaques (860, 820) ;
dans lequel ledit élément capillaire (910) transporte ladite composition volatile dudit réservoir (810) à ladite première plaque (860), et dans lequel ledit actionneur piézoélectrique (840), lors de la réception d'un signal électrique, déplace au moins un micro-bouchon d'une position en place dans au moins une desdites perforations (880) et atomise ladite composition volatile.

**12.** Procédé d'atomisation d'une composition volatile, ledit procédé comprenant les étapes consistant à :

fournir une composition volatile ayant une viscosité supérieure à 5 cP, dans lequel ladite composition volatile est contenue dans un réservoir (810) ;
fournir une unité ultrasonore de micro-bouchons comprenant
une première plaque (860) comprenant une pluralité de perforations (880) s'étendant à travers ladite première plaque (860),
une deuxième plaque (820) comprenant une pluralité de micro-bouchons (870) s'étendant longitudinalement de l'extrémité de base à une extrémité de bout, et conçue pour s'accoupler avec au moins une parmi ladite pluralité de perforations (880),
un actionneur piézoélectrique (840) en communication avec une ou plusieurs desdites première et deuxième plaques (860, 820) ;
fournir un élément capillaire (910) pour transporter ladite composition volatile dudit réservoir (810) à ladite première plaque (860) de ladite unité ultrasonore de micro-bouchons ; et
actionner ledit actionneur piézoélectrique (840) pour atomiser la composition volatile à partir de ladite unité de micro-bouchons.

**13.** Procédé selon la revendication 12, dans lequel l'alimentation électrique est distribuée d'une source d'alimentation audit actionneur piézoélectrique d'une manière permettant de fournir une production intermittente desdites gouttelettes.

**14.** Procédé selon la revendication 12, dans lequel ladite composition volatile a une viscosité supérieure à 10 centipoises, de préférence supérieure à 11 centipoises.

**15.** Procédé selon la revendication 12, comprenant en outre une fourniture d'un élément d'assistance de diffusion pour assister l'atomisation de ladite composition volatile, ledit élément d'assistance de diffusion est choisi parmi : un ventilateur, un élément chauffant, et des combinaisons de ceux-ci.

FIG 1A

FIG 1B

FIG 1C

FIG 1D

FIG. 1E

FIG. 1F

FIG. 1H

FIG. 1G

FIG. 2

## REFERENCES CITED IN THE DESCRIPTION

### Patent documents cited in the description

- US 5518179 A, Humberstone
- US 5297734 A
- US 5657926 A
- US 6378780 B
- US 20110266359 A
- US 7223361 B
- US 20050124512 A

### Non-patent literature cited in the description

- **A. LEO.** Comprehensive Medicinal Chemistry. Pergamon Press, 1990, vol. 4, 295